# EUROPÄISCHE PATENTSCHRIFT

(11) **EP 1 198 459 B1**
(45) Veröffentlichungstag und Bekanntmachung des Hinweises auf die Patenterteilung: **06.05.2004**
(21) Anmeldenummer: 00956270.3
(22) Anmeldetag: 25.07.2000
(51) Int. Cl.: C07D 251/00

(54) **VERFAHREN ZUR HERSTELLUNG VON MELAMIN**
METHOD OF PRODUCING MELAMINE
PROCEDE DE PREPARATION DE MELAMINE

(30) Priorität: 27.07.1999 AT 129999
(43) Veröffentlichungstag der Anmeldung: 24.04.2002
(73) Patentinhaber: AMI Agrolinz Melamine International GmbH, 4020 Linz (AT)
(72) Erfinder: COUFAL, Gerhard, A-4060 Leonding (AT)
(74) Vertreter: Gross, Felix
(86) Internationale Anmeldenummer: PCT/EP2000/007093
(87) Internationale Veröffentlichungsnummer: WO 2001/007421

(56) Entgegenhaltungen:
- EP-A- 0 018 696
- US-A- 3 547 919
- US-A- 3 637 686
- US-A- 4 565 867

## Beschreibung

Die Erfindung betrifft ein Verfahren zur Herstellung von Melamin, bei dem eine NH₃-hältige Melaminschmelze in einem Wirbelschichtapparat verfestigt und aus dem im wesentlichen aus NH₃ bestehenden Kreislaufgas darin enthaltenes Melamin durch Zusatz von NH₃ abgeschieden wird.

Melamin wird bevorzugt durch Pyrolyse von Harnstoff hergestellt, wobei sowohl Niederdruckverfahren als auch Hochdruckverfahren, wie sie beispielsweise in "Ullmann's Encyclopedia of Industrial Chemistry, Vol A 16, 5^{th} ed (1990), Seiten 171-185" beschrieben sind, zur Anwendung kommen können. Das bei der Melaminsynthese anfallende Melamin enthält je nach Herstellverfahren etwa 94-98 Gew.% Melamin, sowie insbesondere Melam, Melem, Ureidomelamin, Ammelin und Ammelide als wesentliche Nebenprodukte bzw. Verunreinigungen und muß für anspruchsvollere Anwendungsgebiete durch besondere Verfahrensschritte weiter gereinigt werden. Dabei ist es wesentlich, daß zur Verhinderung der Rückbildung von Nebenprodukten, bei höheren Temperaturen immer ein hoher NH₃-Druck aufrecht erhalten wird.
Um Melamin in fester Form zu erhalten, kann die flüssige Melaminschmelze beispielsweise gemäß US 4,565,867 mit Ammoniak, oder gemäß PCT/EP99/00353 in einem Wirbelbett mit kalten festen Inertstoffen oder festem Melamin abgekühlt werden. Das Wirbelbett wird bevorzugt mit NH₃ als Kreislaufgas betrieben. Das verfestigte Melamin kann entweder aus dem unteren Teil des Wirbelschichtapparates aufgrund seiner Schwerkraft ausgetragen werden, oder es wird mit dem Kreislaufgas nach oben ausgetragen und beispielsweise über Zyklone und Filter abgeschieden. Das Kreislaufgas muß vor der Rückführung in den Wirbelschichtapparat beispielsweise in einem Wärmetauscher gekühlt werden, wobei sich im Kreislaufgas enthaltenes Melamin an den kalten Flächen des Wärmetauschers abscheidet und diesen verstopfen kann (fouling). Zur Reinigung des Wärmetauschers muß entweder die Apparatur abgestellt werden, oder es ist ein parallel angeordneter Wärmetauscher erforderlich, auf den während der Reinigung umgeschaltet werden kann.
Es war demnach erstrebenswert, eine Möglichkeit zu finden, um diesen zeitlichen, bzw. apparativen Mehraufwand auszuschalten. Unerwarteterweise kann dieses Problem dadurch gelöst werden, daß dem Kreislaufgas kaltes NH₃ zugesetzt wird, wobei das darin enthaltene Melamin teilweise ausgeschieden wird.

Gegenstand der vorliegenden Erfindung ist demnach ein Verfahren zur Herstellung von Melamin, dadurch gekennzeichnet, daß eine aus einem Melamin-Hochdruckreaktor kommende, NH₃-hältige Melaminschmelze,
a) gegebenenfalls nach Abtrennen der Offgase und Aging,
b) gegebenenfalls mit oder ohne NH₃-Zufuhr auf eine Temperatur, die etwa 1 bis 50 °C über dem vom jeweiligen NH₃-Druck abhängigen Schmelzpunkt des Melamins liegt, gekühlt wird,
c) in einen im wesentlichen mit NH₃ als Kreislaufgas betriebenen Wirbelschichtapparat entspannt und mit festem Melamin oder festen Inertstoffen gekühlt und verfestigt wird,
d) das feste Melamin aus dem Wirbelschichtapparat ausgetragen und gegebenenfalls bei einem NH₃-Druck von etwa 10 bis 300 bar und einer Temperatur, die zwischen etwa 150 °C und dem vom jeweiligen NH₃-Druck abhängigen Schmelzpunkt des Melamins liegt, verweilen gelassen wird und
e) anschließend in beliebiger Reihenfolge weiter gekühlt und weiter entspannt wird,
   wobei
f) dem Kreislaufgas, das gegebenenfalls über Zyklone und/oder Filter vorgereinigt wird, kaltes NH₃, bevorzugt flüssiges NH₃ zugesetzt wird, wodurch sich festes Melamin abscheidet.

Melamin bzw. die NH₃-hältige Melaminschmelze wird bevorzugt bei etwa 50 - 800 bar und etwa 325 bis 450 °C in einem Melamin-Hochdruckreaktor aus Harnstoff gebildet, anschließend werden die im wesentlichen aus NH₃, CO₂ und Melamindampf bestehenden Offgase abgetrennt, in einem Harnstoffwäscher vom Melamin befreit und in eine Harnstoffanlage rückgeführt. Nach Abtrennen der Offgase kann die Melaminschmelze beispielsweise mit NH₃ gestrippt werden, wodurch vor allem restliches CO₂ entfernt wird. Zur Erzielung einer besonders guten Melaminqualität ist es vorteilhaft, die flüssige Melaminschmelze unter Ammoniakdruck in einem Aging-Behälter oder einem Röhrenreaktor verweilen zu lassen. Der Druck beim Aging liegt im Bereich von etwa 50 bis 1000 bar, bevorzugt bei etwa 80 bis 600 bar, besonders bevorzugt bei etwa 130 - 400 bar.

Anschließend kann gegebenenfalls mit oder ohne NH₃-Zufuhr auf eine Temperatur, die etwa 1 bis 50 °C, bevorzugt etwa 1 bis 20 °C über dem vom jeweiligen Ammoniakdruck abhängigen Schmelzpunkt des Melamins liegt, gekühlt werden. Dabei ist es vorteilhaft, die Temperatur des flüssigen Melamins beispielsweise durch Zufuhr von flüssigem, gasförmigem oder überkritischem NH₃ abzusenken. Grundsätzlich kann die Temperatur des zu kühlenden flüssigen Melamins in einem großen Bereich variieren. Sie liegt oberhalb des vom jeweiligen Ammoniak-Druck abhängigen Schmelzpunktes von Melamin, üblicherweise jedoch unterhalb von etwa 400 °C, bevorzugt unterhalb von etwa 370 °C, besonders bevorzugt unterhalb von etwa 350 °C. Je höher der Ammoniakdruck ist und je niedriger die Temperatur der Melaminschmelze ist, umso mehr Ammoniak ist im Melamin enthalten, und umso niedriger ist der Schmelzpunkt. Es ist also auch möglich, bei 300 °C und darunter flüssiges Melamin, genauer gesagt, eine Mischung von flüssigem Melamin mit Ammoniak vorliegen zu haben und zu entspannen, falls der Druck hoch genug ist. Besonders vorteilhaft ist es, bei einer Temperatur, die nicht wesentlich über dem jeweiligen Schmelzpunkt des Melamins liegt, in den Wirbelschichtapparat zu entspannen. Die Abkühlung bis knapp oberhalb des Schmelzpunktes des Melamins erfolgt bevorzugt durch Zufuhr von kaltem flüssigem oder gasförmigem, bzw. von überkritischem Ammoniak. Das im flüssigen Melamin enthaltene Ammoniak trägt beim nachfolgenden Entspannen ebenfalls zur Abkühlung bei und wirkt der beim Erstarren des Melamins freiwerdenden Schmelzenthalpie entgegen.
Der NH₃-Druck über der zu kühlenden Melaminschmelze kann in einem großen Bereich variieren. Häufig liegt er beim Druck der im Reaktor durchgeführten Melaminsynthese. Er kann jedoch wesentlich höher liegen, wenn der Melaminsynthese ein "Aging" bei höherem Druck nachgeschaltet ist. Der Druck kann demnach bis zu 1000 bar oder bis zu den ökonomisch und materialmäßig sinnvollen und möglichen Grenzen betragen.

Anschließend wird die NH₃-hältige Melaminschmelze in den Wirbelschichtreaktor eingetragen, auf den dort herrschenden Druck entspannt, abgekühlt und verfestigt. Dies erfolgt beispielsweise gemäß PCT/EP99/00353 in einem mit NH₃ als Kreislaufgas betriebenen Wirbelbett durch Eindüsen der Melaminschmelze und Kühlen mit festem Melamin und/oder mit festen Inertstoffen, beispielsweise Keramik-, Glas- oder Metallpartikeln. Dabei wird die Schmelze über Düsen auf das wirbelnde Gut derart verteilt, daß die Oberfläche der kalten Feststoffteilchen benetzt wird.

Im Falle der Verwendung von festem Melamin als Kühlmedium bzw. als Kristallisationskeime im Wirbelbett erstarrt die Schmelze an deren Oberfläche, wodurch die Melaminteilchen anwachsen und sobald sie eine bestimmte Größe erreicht haben, aufgrund ihres Gewichtes durch eine klassierende Austragsvorrichtung aus der Wirbelschicht abgezogen werden. Durch die Kühlung im Wirbelbett wird bei sehr gutem Wärme- und Stoffübergang ein sehr einheitliches, annähernd kugelförmiges und weitgehend staubfreies Melamingranulat mit guter Rieselfähigkeit erhalten. Um die Zahl der Feststoffpartikel im Wirbelbett konstant zu halten, werden je nach Ausgestaltung des Wirbelbettes und je nach Verfahrensführung laufend neue Feststoffteilchen zugegeben, die als Kristallisationskeime dienen und zu neuem Granulat anwachsen. Es bilden sich aber auch durch einen gewissen Abrieb im Wirbelbett laufend neue Granulat- bzw. Kristallisationskeime, von denen die Granulatbildung ausgehen kann. Die Temperatur des festen Melamins in der Wirbelschicht kann bei jedem beliebigen Wert unterhalb des Schmelzpunktes von Melamin liegen, wobei eine größere Temperaturdifferenz zwischen festem und zu kühlendem, flüssigem Melamin einen größeren Kühleffekt hat.

Im Falle der Kühlung mittels fester Inertstoffe erstarrt das flüssige Melamin an der Oberfläche der Inertstoffe. Einerseits wächst die Schicht an festem Melamin über den Inertstoffen, andererseits wird das aufgewachsene feste Melamin dieser beschichteten Inertstoffpartikel durch das Reiben aneinander ständig abgerieben. Das abgeriebene feste Melamin wird mit dem Wirbelgas ausgetragen und beispielsweise über einen Zyklon abgeschieden.

Die im Wirbelbett vorhandene und aufrechterhaltene Temperatur kann je nach gewählter Verfahrensweise in einem großen Bereich zwischen Raumtemperatur und bis knapp unterhalb des druckabhängigen Schmelzpunktes von Melamin schwanken. Sie beträgt beispielsweise etwa 100 bis etwa 340 °C, bevorzugt etwa 200 bis etwa 340 °C, besonders bevorzugt etwa 280 bis etwa 320 °C. Die Temperatursteuerung im Wirbelbett kann auf mehrfache Weise erfolgen, beispielsweise durch eingebaute Kühlelemente, durch Zufuhr von festem kaltem Melamin, durch gegebenenfalls ausgeschleuste und nach externer Kühlung wieder in das Wirbelbett rückgeführte Inertpartikel, durch Zufuhr von kaltem flüssigem oder gasförmigem NH₃, durch die Temperatur und Menge des Gasstromes, mit dem die Wirbelschicht aufrechterhalten wird und durch die Verdampfungsenthalpie des im flüssigen Melamin enthaltenen Ammoniaks. Die Temperatur des Kreislaufgases kann auch mittels Wärmetauscher, der sowohl zur Kühlung als auch zur Erwärmung des Gasstromes eingesetzt werden kann, reguliert werden. Ein Teil des Ammoniaks wird, zur Kühlung und zur Aufrechterhaltung des Wirbelbettes, im Kreislauf geführt. Der andere Teil des freiwerdenden Ammoniaks kann je nach vorhandenem Druck im Wirbelbett gasförmig oder verflüssigt in den Melamin/Harnstoffprozeß rückgeführt werden.
Der im Wirbelschichtreaktor vorhandene Druck kann je nach gewählter Verfahrensweise ebenfalls in einem großen Bereich schwanken. Er kann von zwischen etwas über 1 bar bis knapp unterhalb des Druckes der zu kühlenden Melaminschmelze betragen. Üblicherweise beträgt der Druck im Wirbelschichtreaktor zwischen etwa 1,5 und etwa 100 bar, bevorzugt zwischen etwa 1,5 bar und 50 bar, besonders bevorzugt zwischen etwa 5 bis 25 bar. Bei einem Druck von über etwa 13 bar kann das überschüssige NH₃-Gas leicht verflüssigt und in die Harnstoff- und Melaminsynthese rückgeführt werden.

Die Temperatur des aus dem Wirbelbett ausgetragenen festen Melamins kann jeden Wert unterhalb des Schmelzpunktes von Melamin betragen.

Das im Wirbelbett erhaltene feste Melamin wird gegebenenfalls bei Temperaturen von bevorzugt etwa 300 °C bis zu seinem vom jeweiligem NH₃-Druck abhängigen Schmelzpunkt und bei Drücken bevorzugt von etwa 20 bis 300 bar während 1 min bis 5 h im Festzustand verweilen gelassen (getempert). Besonders bevorzugt wird dabei so nahe wie möglich, etwa 1 bis 5 °C unterhalb des vom jeweils herrschenden NH₃-Druck abhängigen Schmelzpunktes des Melamins getempert. Das Tempern kann dabei entweder durch längere Verweilzeiten des festen Melamins im Wirbelbett oder in einem separaten, nachgeschalteten Verfahrensschritt erfolgen.

Anschließend wird das feste Melamin gemäß e) in beliebiger Reihenfolge, bevorzugt bis auf Raumtemperatur und Atmosphärendruck weiter gekühlt und entspannt, wobei die Kühlung und Entspannung auch in mehreren Schritten erfolgen kann. Üblicherweise wird zuerst gekühlt und dann entspannt. Für den Fall, daß zuerst entspannt wird, muß zur Verhinderung der Bildung von Nebenprodukten im Anschluß an die Entspannung möglichst rasch und sofort gekühlt werden. Das feste Melamin kann beispielsweise mittels spezieller Kühlelemente, Wärmetauscher, Kühlflächen, Kühlmischer oder Kühlschnecken, wie z.B. Pflugscharmischer, oder Mischer von beispielsweise List, Lödige, Drais oder Buss, gekühlt erden. Die Kühlung kann auch mit flüssigem NH₃ oder mit kalten Gasen, wie z.B. NH₃, Stickstoff oder Luft erfolgen. Weiters ist es möglich, daß die weitere Kühlung und gegebenenfalls weitere Entspannung des festen Melamins gemäß e) in einem im wesentlichen mit NH₃ oder Stickstoff als Kreislaufgas betriebenen Wirbelschichtapparat mit kaltem festem Melamin oder kalten festen Inertstoffen erfolgt, wobei durch Zusatz von kaltem, bevorzugt flüssigem NH₃ zum Kreislaufgas gemäß f) Melamin aus dem Kreislaufgas abgeschieden wird. Dabei können auch die besonderen Vorteile des Wirbelbettes, nämlich die guten Wärme- und Stoffübergänge, für die Kühlung optimal ausgenützt werden. Die Kühlung im Wirbelbett und damit die Einstellung der gewünschten Temperatur im Wirbelbett kann beispielsweise durch eingebaute Kühlelemente oder durch Zufuhr von kaltem flüssigem oder gasförmigem Ammoniak erfolgen. Für den Fall, daß das zu kühlende feste Melamin bereits eine Temperatur von unter etwa 300 °C aufweist, kann zur Kühlung des festen Melamins auch Luft als Kreislaufgas verwendet werden.
Das dem Kreislaufgas gemäß f) zugeführte kalte NH₃ kann gasförmig, überkritisch oder flüssig sein, bevorzugt ist es flüssig. Die Temperatur und Menge des zugeführten gasförmigen oder überkritischen NH₃ kann je nach den gewünschten Bedingungen im Wirbelbett in einem großen Bereich schwanken. Bei Zufuhr von flüssigem NH₃ wirkt vor allem die Verdampfungswärme des NH₃ zur Temperatureinstellung.
Dem Kreislaufgas wird kaltes, bevorzugt flüssiges bzw. superkritisches NH₃ gemäß f) zugesetzt und zwar:
1. entweder vor dem Zyklon, wobei das dabei ausgefallene Melamin über den Zyklon ausgetragen wird,
2. oder zwischen Zyklon und Filter, wobei das dabei ausgefallene Melamin über das Filter abgetrennt wird,
3. oder vor dem Wirbelschichtapparat, wobei das dabei ausgefallene Melamin gemeinsam mit dem Kreislaufgas in die Wirbelschicht rückgeführt wird und als Kristallisationskeim dient.
Es ist aber auch möglich, das NH₃ an 2 beliebigen Stellen oder an allen 3 Stellen gleichzeitig dem Kreislaufgas zuzuführen.

In einer bevorzugten Verfahrensvariante der Erfindung wird eine NH₃-hältige Melaminschmelze in einem Wirbelschichtapparat, der etwa bei 340 °C und 20 bar betrieben wird, mit kaltem festem Melamin gekühlt und verfestigt. Das Wirbelbett wird mit NH₃ als Kreislaufgas (Wirbelgas) betrieben. Die NH₃-hältige Melaminschmelze (etwa 120 bar, 350 °C) wird in das Wirbelbett eingedüst und scheidet sich an den festen Melaminteilchen als Übezug aus verfestigtem Melamin ab. Die schwereren Teilchen werden im unteren Teil des Wirbelschichtapparates abgezogen und bei etwa 340 °C und 20 bar während 20 min im festen Zustand in gesondertem Apparat getempert. Anschließend wird in einem List-Mischer auf etwa unter 80 °C gekühlt, über eine Druckschleuse auf Atmosphärendruck entspannt und überschüssiges NH₃ mit Luft ausgeblasen. Das aus dem Wirbelschichtapparat abgehende Kreislaufgas wird in einem Zyklon vom Großteil der mitgehenen Melamin-Feinanteile und anschließend in einem Filter von den restlichen Melamin-Feinstanteilen befreit. Die Melamin-Fein- und Feinstanteile werden in der Temperstufe mit dem Hauptteil des aus dem Wirbelschichtapparat abgezogenen festen Melamins vereint. Das gereingte, aus dem Filter abgehende Kreislaufgas, das mit Melamindampf beladen, zumeist gesättigt ist, wird über einen Wärmetauscher gekühlt und zur Aufrechteraltung des Wirbelbettes in den Wirbelschichtapparat rückgeführt.

Die Zufuhr von kaltem NH₃ ins Kreislaufgas bewirkt, daß im Kreislaufgas enthaltenes gasförmiges Melamin durch das Abkühlen desublimiert und fest wird, wodurch die zur Kühlung des Kreislaufgases notwendigen Wärmetauscher ganz oder teilweise überflüssig werden.

## Patentansprüche

1. Verfahren zur Herstellung von Melamin, **dadurch gekennzeichnet, daß** eine aus einem Melamin-Hochdruckreaktor kommende, NH₃-hältige Melaminschmelze,
a) gegebenenfalls nach Abtrennen der Offgase und Aging,
b) gegebenenfalls mit oder ohne NH₃-Zufuhr auf eine Temperatur, die etwa 1 bis 50 °C über dem vom jeweiligen NH₃-Druck abhängigen Schmelzpunkt des Melamins liegt, gekühlt wird,
c) in einen im wesentlichen mit NH₃ als Kreislaufgas betriebenen Wirbelschichtapparat entspannt und mit festem Melamin oder festen Inertstoffen gekühlt und verfestigt wird,
d) das feste Melamin aus dem Wirbelschichtapparat ausgetragen und gegebenenfalls bei einem NH₃-Druck von etwa 10 bis 300 bar und einer Temperatur, die zwischen etwa 150 °C und dem vom jeweiligen NH₃-Druck abhängigen Schmelzpunkt des Melamins liegt, verweilen gelassen wird, und
e) anschließend in beliebiger Reihenfolge weiter gekühlt und weiter entspannt wird,
wobei
f) dem Kreislaufgas, das gegebenenfalls über Zyklone und/oder Filter vorgereinigt wird, kaltes NH₃, bevorzugt flüssiges NH₃ zugesetzt wird, wodurch sich festes Melamin abscheidet.

2. Verfahren gemäß Anspruch 1, **dadurch gekennzeichnet, daß** die Kühlung im Wirbelbett gemäß c) mit festem Melamin erfolgt, wobei die eingetragene Melaminschmelze an der Oberfläche der festen Melaminteilchen erstarrt, die Melaminteilchen dabei anwachsen und nach Erreichen einer bestimmten Größe aus dem unteren Teil des Wirbelbettes abgezogen werden.

3. Verfahren gemäß Anspruch 1, **dadurch gekennzeichnet, daß** die Kühlung im Wirbelbett gemäß c) mittels fester Inertstoffe erfolgt, wobei sich die eingetragene Melaminschmelze an der Oberfläche der festen Inertstoffpartikel anlagert, erstarrt, die sich bildende feste Melaminschicht anwächst, durch das Aneinanderreihen der beschichteten Inertstoffpartikel ständig abgerieben, das abgeriebene feste Melamin mit dem Kreislaufgas laufend ausgetragen und beispielsweise über einen Zyklon abgeschieden wird.

4. Verfahren gemäß Anspruch 1, **dadurch gekennzeichnet, daß** gemäß f) das kalte, bevorzugt flüssige NH₃ dem Kreislaufgas vor einem Zyklon zugesetzt wird und das dabei ausgefallene Melamin über den Zyklon ausgetragen wird.

5. Verfahren gemäß Anspruch 1, **dadurch gekennzeichnet, daß** gemäß f) das kalte, bevorzugt flüssige NH₃ dem Kreislaufgas zwischen Zyklon und Filter zugesetzt wird und das dabei ausgefallene Melamin über das Filter abgetrennt wird.

6. Verfahren gemäß Anspruch 1, **dadurch gekennzeichnet, daß** gemäß f) das kalte, bevorzugt flüssige NH₃ dem Kreislaufgas vor dem Wirbelschichtapparat zugesetzt wird und das dabei ausgefallene Melamin gemeinsam mit dem gekühlten Kreislaufgas in die Wirbelschicht rückgeführt wird.

7. Verfahren gemäß einem der Ansprüche 1 bis 6, **dadurch gekennzeichnet, daß** die weitere Kühlung des festen Melamins gemäß e) über Kühlflächen, Kühlschnecken oder Wärmetauscher erfolgt.

8. Verfahren gemäß einem der Ansprüche 1 bis 6, **dadurch gekennzeichnet, daß** die weitere Kühlung des festen Melamins gemäß e) mit flüssigem NH₃ oder mit kalten Gasen, bevorzugt NH₃, Stickstoff oder Luft erfolgt.

9. Verfahren gemäß einem der Ansprüche 1 bis 6, **dadurch gekennzeichnet, daß** die weitere Kühlung und gegebenenfalls weitere Entspannung des festen Melamins gemäß e) in einem im wesentlichen mit NH₃ als Kreislaufgas betriebenen Wirbelschichtapparat mit kaltem festem Melamin oder kalten festen Inertstoffen erfolgt, wobei durch Zusatz von kaltem, bevorzugt flüssigem NH₃ zum Kreislaufgas gemäß f) Melamin aus dem Kreislaufgas abgeschieden wird.

## Claims

1. Process for preparing melamine, **characterized in that** an NH₃-containing melamine melt coming out of a high-pressure melamine reactor
a) where appropriate after removal of the off-gases and ageing,
b) where appropriate with or without supply of NH₃ is cooled to a temperature which is above the melting point of the melamine, which depends on the prevailing pressure of NH₃, by from about 1 to 50°C,
c) in a fluidized-bed apparatus essentially operated using NH₃ as circulating gas, is depressurized and is cooled and solidified using solid melamine or inert solid substances,
d) the solid melamine is discharged from the fluidized-bed apparatus and where appropriate at an NH₃ pressure of from about 10 to 300 bar and at a temperature which lies between about 150°C and the melting point of the melamine, which depends on the prevailing pressure of NH₃, is allowed to age and
e) is then, in any desired sequence, further cooled and further depressurized,
where
f) cold NH₃, preferably liquid NH₃, is added to the circulating gas, which where appropriate is prepurified by way of cyclones and/or filters, the result being that solid melamine precipitates.

2. Process according to Claim 1, **characterized in that** the cooling in the fluidized bed according to c) takes place using solid melamine, where the melamine melt introduced hardens on the surface of the solid melamine particles, whereupon the melamine particles grow and on reaching a particular size are drawn off from the lower part of the fluidized bed.

3. Process according to Claim 1, **characterized in that** the cooling in the fluidized bed according to c) takes place by means of inert solid substances, where the melamine melt introduced deposits on the surface of the particles of inert solid substance and hardens and the solid melamine layer which is formed grows and is constantly abraded by the mutual friction of the coated particles of inert substance and the abraded solid melamine is continuously discharged with the circulating gas and, for example, precipitated by way of a cyclone.

4. Process according to Claim 1, **characterized in that** according to f) the cold, preferably liquid NH₃ is added to the circulating gas prior to a cyclone and the resultant precipitate of melamine is discharged by way of the cyclone.

5. Process according to Claim 1, **characterized in that** according to f) the cold, preferably liquid NH₃ is added to the circulating gas between cyclone and filter and the resultant precipitate of melamine is removed by way of the filter.

6. Process according to Claim 1, **characterized in that** according to f) the cold, preferably liquid NH₃ is added to the circulating gas prior to the fluidized-bed apparatus and the resultant precipitate of melamine, together with the cooled circulating gas, is returned into the fluidized bed.

7. Process according to any of Claims 1 to 6, **characterized in that** the further cooling of the solid melamine according to e) takes place by way of cooling surfaces, cooling screws or heat exchangers.

8. Process according to any of Claims 1 to 6, **characterized in that** the further cooling of the solid melamine according to e) takes place using liquid NH₃ or using cold gases, preferably NH₃, nitrogen or air.

9. Process according to any of Claims 1 to 6, **characterized in that** the further cooling and where appropriate further depressurization of the solid melamine according to e) takes place in a fluidized-bed apparatus operated essentially using NH₃ as circulating gas, using cold solid melamine or cold inert solid substances, where addition of cold, preferably liquid NH₃ to the circulating gas according to f) precipitates melamine from the circulating gas.

## Revendications

1. Procédé de préparation de mélamine, **caractérisé en ce que** l'on procède au refroidissement d'une masse en fusion de mélamine, contenant du NH₃, provenant d'un réacteur à haute pression de mélamine,
a) le cas échéant, après séparation des gaz de rejet et vieillissement,
b) le cas échéant, avec ou sans apport de NH₃, à une température qui se situe environ de 1 à 50°C au-dessus du point de fusion de la mélamine, qui dépend de la pression de NH₃ correspondante,
c) à sa détente dans un appareil à couche fluidisée fonctionnant pour l'essentiel à l'aide de NH₃ en tant que gaz de circulation et à son refroidissement et à sa solidification à l'aide de mélamine solide ou de substances inertes solides,
d) à l'évacuation hors de l'appareil à couche fluidisée de la mélamine solide et à sa mise au repos, le cas échéant, à une pression de NH₃ d'environ 10 à 300 bars et à une température qui se situe entre environ 150°C et le point de fusion de la mélamine, qui dépend de la pression de NH₃ correspondante et
e) en séquence quelconque, à son refroidissement et à sa détente supplémentaires,
f) du NH₃ froid, de préférence, du NH₃ fluide, étant ajouté au gaz de circulation, qui est soumis, le cas échéant, à une purification préalable par l'intermédiaire de cyclones et/ou de filtres, à la suite de quoi la mélamine solide se dépose.

2. Procédé selon la revendication 1, **caractérisé en ce que** le refroidissement dans le lit fluidisé se fait, selon c), à l'aide de mélamine solide, la masse en fusion de mélamine introduite se solidifiant sur la surface des particules de mélamine solides, les particules de mélamine croissant en l'occurrence, et étant évacuées de la partie inférieure du lit fluidisé après avoir atteint une taille déterminée.

3. Procédé selon la revendication 1, **caractérisé en ce que** le refroidissement dans le lit fluidisé se fait, selon c), à l'aide de substances inertes solides, la masse en fusion de mélamine introduite se déposant sur la surface des particules de substances inertes solides et se solidifiant, la couche de mélamine solide qui se forme croissant, la mélamine solide arrachée par la friction réciproque des particules de substances inertes revêtues étant évacuée en permanence à l'aide du gaz de circulation et étant séparée par dépôt, par exemple, par l'intermédiaire d'un cyclone.

4. Procédé selon la revendication 1, **caractérisé en ce que**, selon f), du NH₃ froid, de préférence, fluide, est ajouté avant le cyclone au gaz de circulation et **en ce que** la mélamine ainsi déposée est évacuée par l'intermédiaire du cyclone.

5. Procédé selon la revendication 1, **caractérisé en ce que**, selon f), du NH₃ froid, de préférence, fluide, est ajouté au gaz de circulation entre le cyclone et le filtre et **en ce que** la mélamine ainsi déposée est séparée par l'intermédiaire du filtre.

6. Procédé selon la revendication 1, **caractérisé en ce que**, selon f), du NH₃ froid, de préférence, fluide, est ajouté au gaz de circulation avant l'appareil à couche fluidisée et **en ce que** la mélamine ainsi déposée est reconduite dans la couche fluidisée, conjointement au gaz de circulation refroidi.

7. Procédé selon l'une quelconque des revendications 1 à 6, **caractérisé en ce que** le refroidissement supplémentaire de la mélamine solide se fait, selon e), par l'intermédiaire de surfaces réfrigérantes, de serpentins de réfrigération ou d'échangeurs thermiques.

8. Procédé selon l'une quelconque des revendications 1 à 6, **caractérisé en ce que** le refroidissement supplémentaire de la mélamine solide se fait, selon e), à l'aide de NH₃ fluide ou à l'aide de gaz froids, de préférence, de NH₃, d'azote ou d'air.

9. Procédé selon l'une quelconque des revendications 1 à 6, **caractérisé en ce que** le refroidissement supplémentaire et, le cas échéant, une détente supplémentaire de la mélamine solide se fait, selon e), dans un appareil à couche fluidisée, fonctionnant pour l'essentiel à l'aide de NH₃ en tant que gaz de circulation, avec de la mélamine solide froide ou des substances inertes solides froides, la mélamine étant déposée hors du gaz de circulation, selon f), grâce à l'addition de NH₃ froid, de préférence, fluide, au gaz de circulation.
